**Europäisches Patentamt**

**European Patent Office**

**Office européen des brevets**

(11) Publication number: **0 105 149**
**B1**

## (12) EUROPEAN PATENT SPECIFICATION

(45) Date of publication of patent specification: **16.05.90**

(21) Application number: **83108091.6**

(22) Date of filing: **16.08.83**

(51) Int. Cl.⁵: **C 12 N 15/00, C 12 P 21/02, C 12 N 1/18 // C12R1/865**

(54) Recombinant plasmid containing hepatitis B virus gene, yeast transformed with said recombinant plasmid, and production of hepatitis B virus surface antigen.

(30) Priority: **16.08.82 JP 142460/82**
**20.08.82 JP 145093/82**

(43) Date of publication of application:
**11.04.84 Bulletin 84/15**

(45) Publication of the grant of the patent:
**16.05.90 Bulletin 90/20**

(84) Designated Contracting States:
**AT BE CH DE FR GB IT LI NL SE**

(56) References cited:
**EP-A-0 072 318**
**EP-A-0 073 657**
**EP-A-0 100 561**
**NATURE, vol. 298, no. 5872, 22nd July 1982, pages 347-350, Chesham, Bucks, GB; P. VALENZUELA et al.: "Synthesis and assembly of hepatitis B virus surface antigen particles in yeast"**

**The file contains technical information submitted after the application was filed and not included in this specification**

(73) Proprietor: **Science and Technology Agency, Minister's Secretariat, Director of Finance Division**
**2-1, Kasumigaseki 2-chome**
**Chiyoda-ku Tokyo-to (JP)**

(72) Inventor: **Miyanohara, Atsushi**
**8-16, Kori-motomachi**
**Neyagawa-shi Osaka-fu (JP)**
Inventor: **Nozaki, Chikateru**
**622-8, Shinchi Shimizu-machi**
**Kumamoto-shi Kumamoto-ken (JP)**
Inventor: **Hamada, Fukusaburo**
**2679-2, Suya Nishigochi-machi**
**Kikuchi-gun Kumamoto-ken (JP)**
Inventor: **Toh-e, Akio**
**2-2-1, Mitaki-hommachi Nishi-ku**
**Hiroshima-shi Hiroshima-ken (JP)**
Inventor: **Ohtomo, Nobuya**
**6-22-29, Shimasaki**
**Kumamoto-shi Kumamoto-ken (JP)**
Inventor: **Matsubara, Kenichi**
**3-1-57-909, Fukushima Fukushima-ku**
**Osaka-shi Osaka-fu (JP)**

(74) Representative: **Vossius & Partner**
**Siebertstrasse 4 P.O. Box 86 07 67**
**D-8000 München 86 (DE)**

# EP 0 105 149 B1

(56) References cited:

PROCEEDINGS OF THE NATIONAL ACADEMY OF SCIENCES USA, vol. 80, no. 1, January 1983, pages 1-5, Washington, US; A. MIYANOHARA et al.: "Expression of hepatitis B surface antigen gene in yeast"

EXPERIENTIA, vol. 38, June 1982, p. 745

Nature, vol. 298, July 1982, p. 347-350

Current Topics in Microbiology and Immunology vol. 96, (1982), p. 101-117

**Description**

The present invention relates to a novel recombinant plasmid comprising a Hepatitis B virus DNA sequence and its use in a process for the production of Hepatitis B virus surface antigen subtype adr. More particularly, it relates to a recombinant plasmid comprising a DNA sequence coding for the Hepatitis B virus (HBV) surface antigen subtype adr (hereinafter referred to as "HBs antigen", "HBsAg" or "s antigen") which can replicate both in *Escherichia coli* and in yeast and to its use in a process for the production of an immunologically active HBs antigen (HBsAg) comprising culturing e.g. a transformed yeast in an appropriate medium preferably under such conditions that the acid phosphatase promoter is not repressed.

Hepatitis B is usually caused by transfusing blood of HBV positive patients. There is no suitable drug for complete remedy. The most suitable prophylaxis is a vaccine containing the HBsAg. However, it is very difficult to produce the HBsAg vaccine in a technical scale, because HBV is infectious only to humans and chimpanzees (one has not succeeded to infect a cell culture with HBV), and owing to this specificity of HBV, HBsAg can be obtained only from human donors.

It is known to prepare HBsAg by recombinant DNA technology in *E. coli* as host organism. (cf. Japanese Patent Laid Open Application No. 104887/1980). However, according to this method using *E. coli*, it is still difficult to produce the desired HBsAg in a large scale, because the produced HBsAg is easily degraded within *E. coli* and further growth of *E. coli* is inhibited by the HBsAg produced. This results in a low productivity of HBsAg.

In Experientia, Vol. 38 (June 1982), No. 6, page 745, a summary of the genetic analysis of the acid phosphatase promoter is given. Furthermore it is stated that the phosphatase promoter is used for the expression of foreign genes in yeast. A. Hinnen and B. Meyhack, Vectors for Cloning in Yeast, Current Topics in Microbiology and Immunology, Vol. 96 (1982), pages 101—117, generically disclose plasmid hybrids between *E. coli* plasmid DNA and yeast DNA which are used for yeast transformation. P. Valenzuela et al reported the production of HBsAg particles in yeast (Nature, *298*, 347—350; 22 July, 1982). DNA sequence coding for HBsAg is inserted into an *E. coli*-yeast shuttle vector. The alcohol dehydrogenase I (AdHI) expression control region is used to achieve the expression of the HBsAg structural gene which is located downstream of the promoter. According to this method, however, the obtained amount of HBsAg is very small. This method, therefore, is not satisfactory for the production of the desired HBsAg in large scale.

The technical problem underlying the present invention is to provide a novel recombinant plasmid which can produce effectively HBsAg in a large scale. This problem is solved by recombinant plasmid for use in the production of the Hepatitis B virus surface antigen comprising the following components:

(a) DNA sequences of the yeast *Saccharomyces cerevisiae* including ars 1, the 2 μ ori, a marker gene for a transformed yeast permitting the synthesis of leucine, histidine, tryptophane, uracil or adenine by the transformant;

(b) a DNA sequence of the *E. coli* plasmid pBR322 which has a size of about 3.7 kb and which includes a marker gene for a transformed *E. coli* encoding a resistance for ampicillin as well as the origin of replication;

(c) the expression control region of the repressible acid phosphatase gene of yeast without the entire phosphatase structural gene and without a region in the range of from +1 (ATG) to −100 bp upstream of the structural gene; and

(d) a Hepatitis B virus DNA sequence under the control of the phosphatase promoter of (c) which Hepatitis B virus DNA sequence includes a fragment of 1.3 kb containing a DNA sequence coding for the 226 amino acids of the Hepatitis B virus surface antigen of the subtype adr.

The novel recombinant plasmid containing a HBV DNA sequence coding for the 226 amino acids of the HBsAg subtype adr is obtained by utilizing a shuttle vector which contains both DNA sequences of the *E. coli* plasmid pBR322 and DNA sequences of *Saccharomyces cerevisiae* and the expression control region of the repressible acid phosphatase gene of yeast. This vector can replicate in both host organisms. The DNA sequence coding for said HBsAg is inserted into the vector under the control of the acid phosphatase promoter. The entire acid phosphatase structural gene and the region in the range of from +1 (ATG) to −100 bp upstream of the phosphatase structural gene are deleted. A transformed yeast is prepared by transforming the yeast with the thus obtained recombinant plasmid. The desired HBsAg can be produced by cultivating the transformed yeast in an appropriate nutrient medium, preferably under such conditions that the acidic phosphatase promoter is not repressed.

The recombinant plasmid, the transformed yeast and the production of HBsAg with the transformed yeast will be illustrated below in more detail.

(1) Isolation of the HBV DNA sequence from the HBV genome

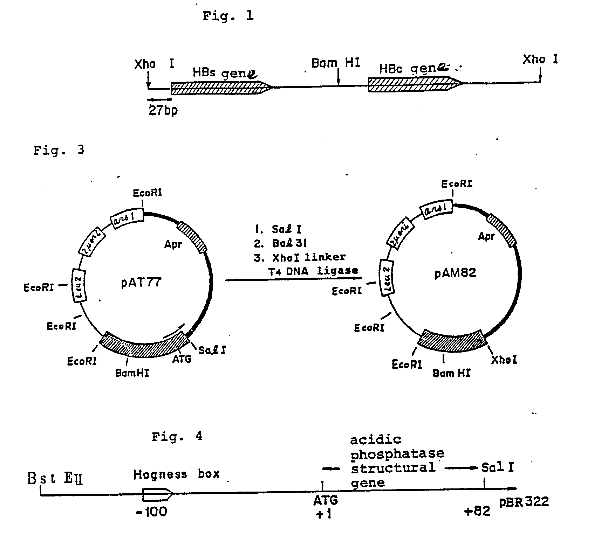

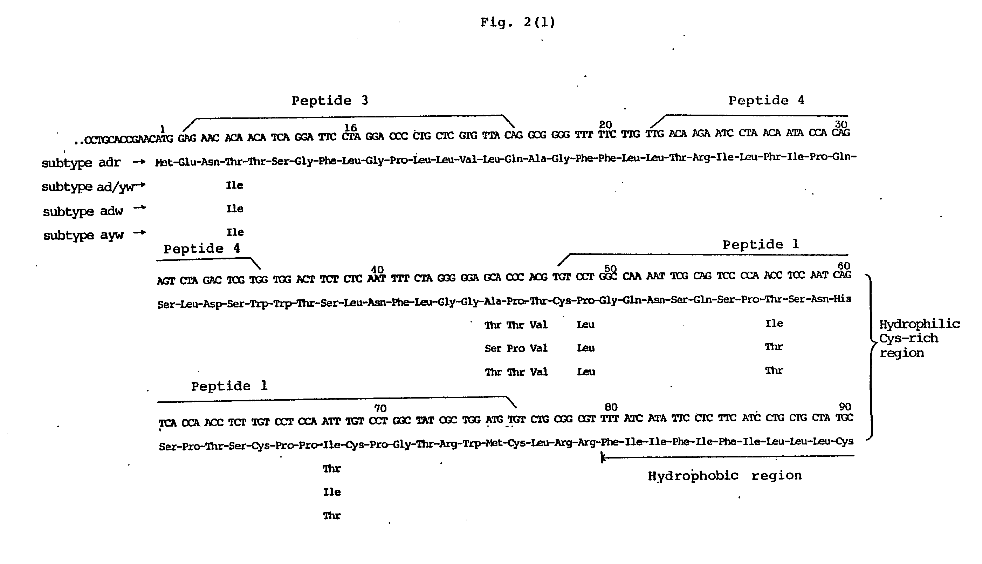

The HBV DNA sequence to be inserted into the shuttle vector is a genomic DNA sequence of HBV subtype adr which is frequently observed in Japan and also in countries of Southeast Asia and is cloned in *E. coli*. The genomic HBV DNA (HBV genome) contains a unique recognition site for the restriction enzymes Xho I and BamHI. The fragment, e.g. a fragment having an Xho I site at the terminus, which is obtained by cleaving the HBV genome with Xho I has the structure as shown in Figure 1, wherein the HBs coding sequence and the HBc coding sequence are present in the same orientation.

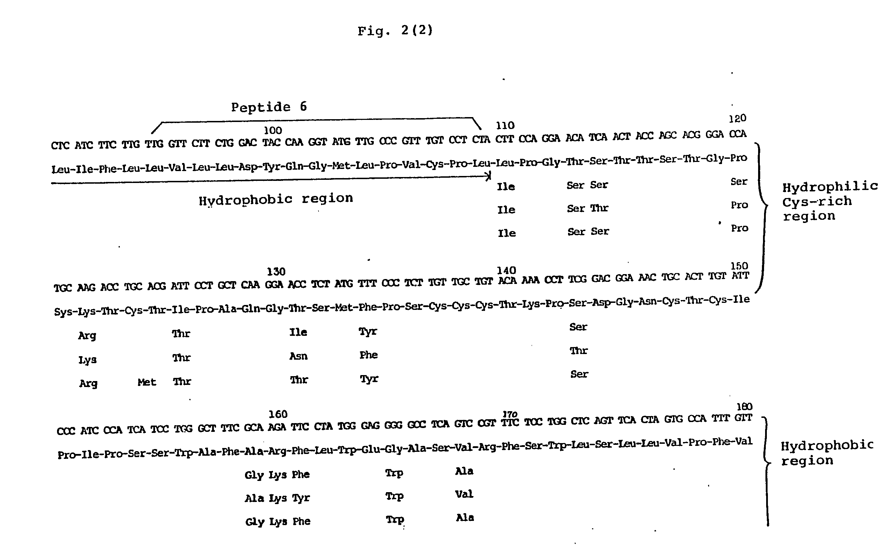

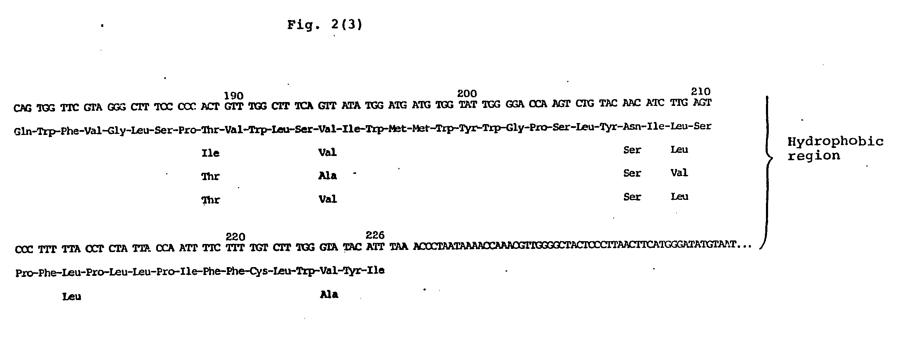

As is shown in *Figure 1*, the HBs coding sequence starts at the 28th nucleotide from the 5'-end of the restriction endonuclease site Xho I and contains a nucleotide sequence corresponding to a polypeptide consisting of 226 amino acids and also an HBc coding sequence downstream thereof in the same orientation. When the genomic DNA of HBV (3.2 kb) linearized with Xho I is cleaved with BamHI, two fragments are obtained, i.e. a fragment (about 1.3 kb) containing the HBs coding sequence and a fragment (about 1.9 kb) containing the HBc coding sequence. The nucleotide sequence of the HBs coding sequence is shown in *Figure 2*. The DNA sequence of HBV subtype adw which is frequently observed in European countries and U.S.A. is already known. Both the HBs and the HBc coding sequences do not contain any intervening sequence. In *Figure 2*, the upper line shows the nucleotide sequence of the region coding for the surface antigen and the second line shows the amino acid sequence encoded thereby. The numbers above the nucleotide sequence show the number of amino acids counted from the N-terminus of the surface antigen. The three lines below designate the data of the ad/yw type (analyzed by Pasek et al., Nature *282*, 575—579, 1979), the adw type (analyzed by P. Valenzuela et al., Nature, *280*, 815—819, 1979) and the ayw type (analyzed by Charnay et al., Proc. Natl. Acad. Sci., U.S.A. *76*, 2222—2226, 1979) respectively.

The HBV DNA sequence is prepared as follows:

Viral particles (Dane particles) are isolated from blood of a person who is positive for HBsAg. The HBV DNA sequence (3,200 bp) usually is a partially double-stranded circular DNA. Between 15 to 50% of the HBV DNA sequence is single-stranded. Accordingly, in order to change the single-stranded region to a double-stranded DNA sequence suitable for cloning, the HBV DNA sequence is treated with an endogenous DNA polymerase by the method of Sattler and Robinson, Journal of Virology, *32*, 226—233, 1979. After filling in the partially single stranded DNA, the double-stranded DNA is extracted, and propagated by cloning in *E. coli*. This cloned double-stranded HBV DNA sequence is treated with an appropriate restriction enzyme to give a fragment which is used for the construction of the desired plasmid.

The genomic HBV DNA sequence used is of subtype adr.

(2) Shuttle vector

The shuttle vector used in the present invention is a plasmid vector which contains both DNA sequences of the yeast *Saccharomyces cerevisiae* and DNA sequences of the *E. coli* plasmid pBR322 and furthermore the expression control region of the repressible acid phosphatase gene of yeast, e.g. *Saccharomyces cerevisiae*.

The yeast derived DNA sequence usually contains a DNA sequence which is necessary for replication of a plasmid in the yeast as an episome, for instance, a DNA sequence necessary for the autonomous replication in yeast (which is designated "ars 1"), and a DNA sequence necessary for the replication of 2 μm DNA (which is designated "2 μ ori"), and contains optionally a gene useful as a selective marker for the transformed yeast. The selective marker includes, for example, a leucine-producing gene, a histidine-producing gene, a tryptophane-producing gene, a uracil-producing gene, an adenine-producing gene, or the like, which may be used alone or in combination of two or more thereof.

The pBR322 derived DNA sequences contain a DNA sequence necessary for the application of the plasmid in *E. coli*, for example, a DNA sequence of a replication initiating region of the plasmid Col EI, and a marker gene encoding ampicillin resistance for the transformed *E. coli*.

The shuttle vector used in the present invention is characteristic in that it carries the repressible acid phosphatase promoter of yeast. This acid phosphatase promoter is usually a promoter controlling the expression of a polypeptide of 60,000 daltons (p60) which constitutes the acid phosphatase.

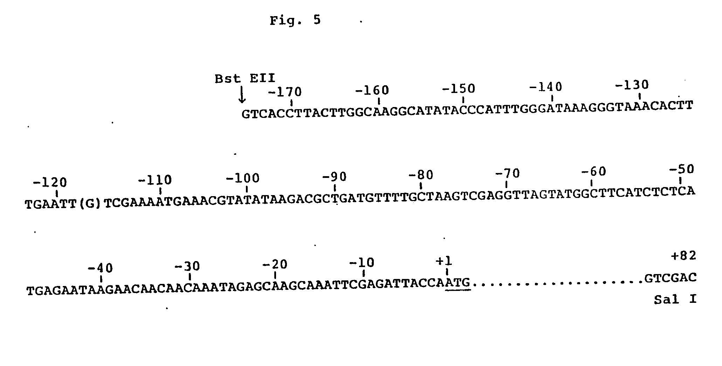

A representative example of the shuttle vector is a vector which is prepared by recombining a yeast derived DNA containing the ars 1, 2 μ-ori and a leucine producing gene (leu 2) with the *E. coli* plasmid pBR322. The shuttle vector is designated "pAT77" (Fig. 3) and is prepared as follows.

An EcoRI fragment of about 8,000 base pairs (8 kb) containing a DNA sequence coding for a polypeptide (p60) of 60,000 daltons which constitutes the repressible acid phosphatase (cf. PNAS, *77*, 6541—6545, 1980, and PNAS, *79*, 2157—2161, 1982) is inserted into the EcoRI site of the *E. coli* plasmid pBR322 (cf. Sutcliffe, J. G.; Cold Spring Harbor Symposium, *43*, 77—90, 1979) to give a plasmid, which is used as the starting material. Said EcoRI fragment (8 kb DNA fragment) contains a unique recognition site for the restriction enzyme Sal I at a position where it is cleaved into a fragment of 2.8 kb and a fragment of about 5.2 kb. The ampicillin-resistance gene of pBR322 is located upstream of the 2.8 kb fragment.

The starting plasmid is digested with the restriction enzyme Sal I and re-annealed with T4 DNA ligase to give a plasmid which has lost the 5.2 kb acid phosphatase gene fragment. This plasmid is designated "pAT 25". pAT 25 is a plasmid consisting of a 3.7 kb fragment extending from the EcoRI site to the Sal I site of pBR322 which contains the ampicillin-resistance gene and a fragment (about 2.8 kb) extending from the EcoRI site to the Sal I site of the yeast acid phosphatase gene.

An EcoRI fragment (1.4 kb) containing a DNA sequence necessary for the autonomous replication of the ars 1 gene and a trp 1 gene of yeast (cf. PNAS, *76*, 1035—1039, 1979) is inserted into the EcoRI site of pAT 25 in order to obtain plasmid "pAT 26". Said ars 1-trp 1 fragment contains a unique recognition site for the restriction enzyme Hind III within the trp 1 gene.

A Hind III fragment containing the leu 2 gene of yeast and a DNA sequence necessary for the replication

of 2 μm DNA (2 μ ori) is inserted into this unique Hind III site of the recombinant plasmid pAT 26 (cf. Tohe, A. et al., J. Bacteriol., 141, 413—416, 1980) in order to obtain the desired shuttle vector pAT 77 (Fig. 3).

The plasmids pAT 77 and pAM 82 derived therefrom as described hereinafter have the structures as shown in Figure 3. In Figure 3, the dark line represents the Eco RI/Sal I-pBR322 fragment containing the ampicillin resistance gene (Apr) linked to ars 1, 2 μ ori and the leu 2 gene in this order and to the 2.8 kb Eco RI/Sal I-fragment of the acid phosphatase gene. The recombinant plasmid pAT 77 can replicate in E. coli because of the presence of the origin of replication of pBR322 and it can also replicate in yeast because of the presence of the ars 1 and 2 μ ori genes. Moreover, this plasmid contains as markers in order to select for transformed E. coli the ampicillin-resistance gene (Apr) and for transformed yeast the leu 2 gene, and thereby it fulfills the requirements to be used as a shuttle vector.

The shuttle vector is required for propagating the recombinant plasmid in E. coli.

The gene map around the acid phosphatase promoter of the shuttle vector pAT 77 is shown in Figure 4. The nucleotide sequence of the BstEII/Sal I region in this shuttle vector is shown in Figure 5. The ATG codon (methionine) shown in Figures 4 and 5 is the start codon of the acid phosphatase structural gene. More specifically, the repressible about 2.8 kb gene fragment of the acid phosphatase of this vector contains the region covering from about 2.7 kb upstream from the structural gene to the 82nd nucleotide base pair (82 bp) of the structural gene.

The shuttle vector pAT 77 is cleaved with the restriction enzyme Sal I, followed by treating with the exonuclease Bal 31, by which a part of the complete structural gene of the acid phosphatase as shown in Figures 4 and 5 and further optionally various regions upstream therefrom are deleted. This deletion is effected for appropriate regions before the acid phosphatase promoter region: TATATAA (Hogness box), i.e. −100 bp. The regions to be deleted can be controlled by the conditions for treating with the exonuclease Bal 31 and are usually in the range of from +1 to −100 bp, preferably from +1 to −50 bp and most preferably from +1 to −33 bp. The loss of the Hogness box by extensive deletion, i.e. over −100 bp, implies the loss of the control region, resulting in a lower yield of the HBsAg in the cultivated transformed yeast cells. On the other hand, when the deletion is insufficiently effected so that a part of the acid phosphatase structural gene remains, a fusion protein of the HBsAg and a phosphatase peptide may be produced which is not desirable.

After deleting the complete structural gene of the acid phosphatase and some regions upstream thereof, a synthetic or natural linker, for example a Sal I linker or Xho I linker, is recombined thereto to give a circular plasmid, by which there is obtained a shuttle vector in which a foreign gene can be inserted the expression of which is controlled by the acid phosphatase promoter. This shuttle vector can readily be cleaved at a site to be recombined by treating it with a conventional restriction enzyme, such as Sal I or Xho I, and hence, is preferably used in order to recombine it with the desired gene.

(3) Construction of an HBsAg expression plasmid

The recombinant plasmid of the present invention, i.e. a plasmid recombined with an HBsAg coding sequence, is prepared by cleaving the above shuttle vector with a restriction enzyme recognizing the linkers used, for example Sal I or Xho I linkers, and then recombining the resulting cleaved fragment with the HBV DNA sequence as mentioned hereinbefore. The recombinant plasmids thus obtained are propagated in E. coli. The desired plasmid which is recombined in the correct orientation is detected by restriction analysis, with the enzymes Xho I or Sal I (insertion) or EcoRI and Xho I (orientation of the insertion).

(4) Transformation of yeast

The yeast to be transformed is a mutant strain of yeast which is deficient in the selective marker gene of the transformed yeast carried on the plasmid, for example, a leucine-requiring mutant, Saccharomyces cerevisiae AH22 [a, leu 2, his 4, can 1 (Cir⁺)] (cf. A. Hinnen et al., Proc. Natl. Acad. Sci; U.S.A., 75, 2157—2161, 1978). After propagation in E. coli, the recombinant plasmid is introduced into the mutant strain of yeast in the usual manner, for example, by mixing the plasmid DNA with cells obtained by converting them into spheroplasts, followed by treating with Ca⁺⁺, by which the introduction of the plasmid is effected. The desired transformed yeast is selected and isolated from the yeast culture thus treated based on the expression of a gene complemental with the mutation of the host yeast carried on the vector, for example, expression of a leucine-producing gene.

In addition to the above-mentioned leucine-requiring strain, various other mutant strains such as a histidine-requiring strain, tryptophane-requiring strain, uracil-requiring strain, adenine-requiring strain, or the like can be used as the yeast. One example of other strain is S. cerevisiae SHY3 (a, ste-VC9, ura 3, trp 1, leu 2, his 3, ade 1, can 1).

(5) Cultivation of transferred yeast and production of HBsAg.

The transformed yeast obtained above is cultivated in the usual manner in a nutrient medium containing phosphoric acid. The cultivated cells growing in exponential phase are transferred to an inorganic phosphate free medium and are further cultivated: The acid phosphatase promoter is not repressed. After the cultivation the cells are collected and lysed in the usual manner to give a lysed cell solution containing a large amount of the desired HBsAg.

Depending on the type of yeast, for instance, when the Pho 80 mutant strain (cf. Tohe, A. et al., J.

Bacteriol., *145*, 221—232, 1981) is used, the cultivation is not necessarily required to be carried out under such conditions that the acid phosphatase promoter is not repressed, but may be carried out under the usual conditions to give directly the desired HBsAg in a large amount.

The HBsAg thus obtained behaves like the natural HBsAg from human donors in terms of immunological properties, and hence, is useful for the preparation of a HBV vaccine like the HBsAg from human donors.

The present invention is illustrated by the following example but should not be construed to be limited thereto.

Example 1
(1) Preparation of HBV DNA

(i) Preparation of virus DNA

Pooled blood plasma (700 ml) obtained from ten patients who are positive in HBsAg (subtype adr) and HBcAg is centrifuged at 5,000 r.p.m. for 20 minutes to remove undissolved substances. The resulting solution is centrifuged at 4°C, 18,000 r.p.m. for 8 hours, and the resultant precipitate is re-dissolved in 10 ml of a buffer (pH 7.5) of 10 mM Tris-HCl, 0.1 M NaCl and 1 mM EDTA. The solution is added to the top of a centrifuge tube containing 30% sucrose, which is centrifuged at 4°C, 39,000 r.p.m. for 4 hours. The resultant precipitate is redissolved in the same buffer as above.

In order to allow the following operations the buffer solution is subjected to the reaction with HBV DNA polymerase by treating it in a mixture (500 µl) of 67 mM Tris-HCl (pH 7.5), 80 mM $NH_4Cl$, 25 mM $MgCl_2$, 0.5% NP40 (Tergitol®, manufactured by Sigma Co.), 0.1% 2-mercaptoethanol, 330 µM dGTP (deoxycytidine triphosphate), dGTP (deoxyguanosine triphosphate), and dATP (deoxyadenosine triphosphate), 0.5 µM α-[32P]dTTP (deoxythymidine triphosphate) at 37°C for 3 hours. To the reaction mixture is added the same volume of 100 mM EDTA solution. By the above DNA polymerase reaction, the single-stranded region of the DNA is repaired to a completely double-stranded structure which is [32P] labeled. This DNA is added to the top of a centrifuge tube wherein 30%, 20% and 10% aqueous solutions of sucrose are packed in layers in this order, and it is centrifuged at 4°C, 39,000 r.p.m. for 4.5 hours.

In order to digest the proteins strongly bonded to DNA, the precipitates obtained above are treated in a mixture (200 µl) of 1 mg/ml of Pronase E (manufactured by Kaken Kagaku K.K.) and 0.2% aqueous sodium lauryl sulfate solution at 37°C for 2 hours. The resulting mixture is extracted with phenol (200 µl) twice, and the resulting DNA-containing extract is washed with ether to remove the phenol and to give a solution of HBV DNA. The DNA thus obtained has a specific radioactivity of $2.5 \times 10^6$ cpm/µg and can be used for digestion with restriction enzymes.

(ii) Cloning of HBV DNA

The double-stranded circular HBV DNA obtained above is cloned by using λ-phage Charon 16A DNA as a vector and then it is subcloned by using the known plasmid pACYC177 as a vector as follows.

(A) Cloning in the system of λ-phage Charon 16A host-vector:

HBV DNA (20 ng) is treated with endonuclease Xho I in a mixture (20 µl) of 10 mM Tris-HCl (pH 7.4), 7 mM $MgCl_2$, 100 mM NaCl and 7 mM 2-mercaptoethanol at 37°C for 2 hours. The resulting mixture is extracted with phenol (20 µl) and further with ether, and to the aqueous layer is added a double volume of cooled ethanol to precipitate DNA. The mixture is kept at −70°C for one hour and then centrifuged at 10,000 r.p.m. for 5 minutes, and the precipitated DNA is recovered. The precipitate thus separated is dissolved in a mixture (5 µl) of 10 mM Tris-HCl (pH 7.4) and 1 mM EDTA. The HBV DNA and an equimolar amount of λ-phage Charon 16 A DNA (having a unique Xho I recognition site) obtained by cleavage with endonuclease Xho I in the same manner as described above are reacted with T4 DNA ligase [a mixture of 50 mM Tris-HCl (pH 7.4), 10 mM $MgCl_2$, 10 mM dithiothreitol, 100 µg/ml calf serum albumin, 0.5 mM ATP and 0.5 µl enzyme preparation (T4 ligase, manufactuerd by Takara Biomedicals, $1—5 \times 10^3$ unit/ml)] at 4°C for 18 hours). The reaction mixture is extracted with phenol and ether and then subjected to precipitation with ethanol in the same manner as described above. The precipitates thus obtained are dissolved in a mixture (10 µl) of 10 mM Tris-HCl (pH 7.4) and 1 mM EDTA.

The thus annealed DNA is subjected to in vitro packaging operation to form λ-phages in the same manner as described in "Methods in Enzymology", *68*, (1979) 299—309. Furthermore plaques ($10^4$) are formed therefrom on an L-agar plate (23 cm×23 cm) by using *E. coli* DP50-SupF (cf. Blattner, F. R. et al., Science *196*, 161, 1977) as an indicator. These plaques are subjected to plaque hybridization using 32P-labeled HBV DNA prepared above as a probe (cf. Science, *196*, 180, 1977) in order to select plaques formed from the phages containing HBV DNA. A plurality of the desired phages are separated.

(B) Subcloning by using plasmid pACYC177 as a vector:

From HBV DNA cloned in Charon 16A as obtained in step (A) a phage DNA is prepared by using *E. coli* DP50-SupF as bacterium to be infected in the same manner as described in "Methods in Enzymology", *68*, 245—378, 1979. The DNA thus obtained is digested with Xho I under the same conditions as described above for 2 hours, and the resulting reaction mixture is subjected to an electrophoresis in a 0.75% agarose gel to isolate HBV DNA (3.2 kb). The HBV DNA is absorbed onto DEAE (diethylaminoethyl cellulose) paper (manufactured by Toyo Roshi, Japan) in order to separate it from the vector DNA and then eluted with 1 M NaCl aqueous solution to give an HBV DNA having Xho I termini at both ends.

Separately, plasmid pACYC177 (cf. Chang, A. C. Y., Cohen, S. N., J. Bacteriol., *134*, 1141—1156, 1978) having a unique Xho I cleavage site within its kanamycin-resistance gene is digested with Xho I, and the product is purified by phenol extraction, ether treatment and ethanol precipitation in the same manner as described above.

The thus obtained pACYC177 cleaved with Xho I is mixed with XhoI-terminus HBV DNA obtained above in a molar ratio of 1:5, and the mixture is annealed and ligated with T4 DNA ligase for 18 hours as described above.

The annealed DNA preparation (10 μl) obtained above is added to a suspension of *E. coli* (0.1 ml) which is prepared by treating a culture broth of *E. coli* χ1776 [cf. R. Curtiss III et al., "Molecular cloning of recombinant DNA", eds. W. A. Scott and R. Werner, page 99, Academie Press (1977)] by the procedure as described in M. V. Norgard, Gene, *3*, 279 (1979). The mixture is mixed well and allowed to stand at 0°C for 25 minutes. The mixture is applied onto an L-agar plate containing ampicillin (20 μg/ml), α-biotine (1 μ g/ml), diaminopimelic acid (100 μg/ml) and thymine (20 μ g/ml) and is incubated at 37°C overnight. The resulting colonies are applied onto both an agar plate containing kanamycin (20 μg/ml) and an agar plate containing ampicillin (20 μg/ml). The colonies which grow only on the agar plate containing ampicillin are selected. pACYC177 contains an ampicillin-resistance gene and a kanamycin-resistance gene, but when HBV DNA is inserted into the Xho I site of the kanamycin-resistance gene, the kanamycin-resistance is lost. Accordingly, the selected colonies contain a recombinant DNA of pACYC177-HBV DNA. From the colonies thus selected, a plasmid is prepared by the procedure as described by K. Matsubara (J. Virol., *16*, 479, 1975). The plasmid thus obtained, i.e. the recombinant DNA of pACYC177-HBV DNA (which is designated "pHBV"), is treated with Xho I under the same conditions as described above to give a complete HBV DNA fragment (3.2 kb). Besides, when it is treated with Xho I and BamHi, there is obtained a fragment (about 1.3 kb) containing the HBsAg gene.

(2) Preparation of the shuttle vectors pAM81, 82, 83 and 84

An EcoRI fragment of about 8,000 base pairs (8 kb) containing a DNA sequence coding for a polypeptide (p60) of 60,000 daltons which constitutes the repressible acid phosphatase (available from Yeast S288C gene bank, Clarke, L. and Carbon, J. Cell, *9*, 91—99, 1976) is inserted into the EcoRI site of the *E. coli* plasmid pBR322 to give a plasmid, which is used as the starting material.

The starting plasmid is digested with the restriction enzyme Sal I and re-annealed with T4 DNA ligase to give plasmid pAT25 which has lost the 5.2 kb the acid phosphatase gene fragment. Plasmid pAT 25 is a plasmid consisting of a fragment (about 3.7 kb) extending from the EcoRI site to Sal I site of pBR322 which contains the ampicillin-resistance gene and a fragment (about 2.8 kb) extending from the EcoRI site to the Sal I site of the yeast acid phosphatase gene.

An EcoRI fragment (1.4 kb) containing the ars 1 and trp 1 gene which is prepared by treating plasmid YRP 7 (cf. Struhl, K. et al. Proc. Natl. Acad. Sci. U.S.A., *76*, 1035—1039, 1979) with EcoRI is inserted into the EcoRI site of the above pAT 25 in order to obtain plasmid pAT 26. Said ars 1-trp 1 fragment contains a unique recognition site for the restriction enzyme Hind III within the trp 1 gene.

A Hind III fragment containing a leu 2 and 2 μ ori which is prepared by treating the plasmid pSLE 1 (cf. Tohe, A. et al. J. Bacteriol., *141*, 413—416, 1980) with Hind III is inserted into the Hind III site of the above pAT 26 in order to obtain the desired shuttle vector pAT 77. *Saccharomyces cerevisiae* harbouring pAT77 (i.e. *Saccharomyces cerevisiae* AH 22/pAT 77) has been deposited at the Fermentation Research Institute, Agency of Industrial Science and Technology, Japan according to the Budapest Treaty as "FERM BP-324".

The pAT 77 thus obtained (1 μg) is cleaved with Sal I and is then treated with the exonuclease Bal 31 (0.1 U) in a solution (50 μl) of 20 mM Tris-HCl (pH 8.2), 12 mM CaCl₂, 12 mM MgCl₂, 0.2 M NaCl and 1 mM EDTA for 30 seconds to one minute. The reaction mixture is subjected to phenol extraction and ethanol precipitation in the same manner as described above. The resulting precipitates are treated with an Xho I linker (1 pmol) and T4 DNA ligase under the same conditions as described above for 12 hours.

*E. coli* χ1776 is treated with the above reaction mixture by the procedure as described in R. Curtiss III et al., "Molecular cloning of recombinant DNA", eds. W. A. Scott and R. Werner, page 99, Academic Press (1977), so as to transform the *E. coli* χ1776 to give an ampicillin-resistant transformant. From the resulting transformant colonies, plasmid DNAs are prepared by the procedure as described by K. Matsubara (J. Virol., *16*, 479, 1975). According to the Maxam-Gilbert method (cf. Maxam, A. & Gilbert, W., P.N.A.S., *74*, 560—564, 1977), the nucleotide sequence of the resulting DNAs is determined. Furthermore, the region of the acid phosphatase gene deleted with Bal 31 is determined. Among these DNAs, the desired plasmids pAM 81, pAM 82, pAM 83 and pAM 84 which are deficient in the complete structural gene of phosphatase are selected and isolated.

Designating "A" in the codon ATG encoding the first amino acid (methionine) of the product p60 of the phosphatase structural gene as "+1", the following regions are deleted in these shuttle vectors, pAM 81: till +2, pAM 82: till −33, pAM 83: till −50, and pAM 84: till −51. *Saccharomyces cerevisiae* harbouring pAM 81, pAM 82, pAM 83 and pAM 84 (i.e. *Saccharomyces cerevisiae* AH 22/pAM 81, AH 22/pAM 82, AH 22/pAM 83 and AH 22/pAM 84, respectively) have been deposited at the Fermentation Research Institute, Agency of Industrial Science and Technology, Japan according to the Budapest Treaty as "FERM BP-325", "FERM BP-313", "FERM BP-327", and "FERM BP-326", respectively.

(3) Preparation of HBsAg expression plasmids

(i) Preparation of plasmids containing the complete HBV DNA

HBV DNA obtained by treating plasmid pHBV (pACYC 177-HBV DNA) with Xho I is recombined with the Xho I cleaved shuttle vector, pAM 81, pAM 82, pAM 83 or pAM 84 in a molar ratio of 5:1 by annealing and ligating with T4 DNA ligase under the same conditions as described above.

*E. coli* χ1776 is transformed with the reaction mixture and plasmid DNA is prepared from the resulting ampicillin-resistant transformant in the same manner as described hereinbefore. The DNAs thus prepared are analyzed with various restriction enzymes, such as Xho I, Xba I and Hind III, and thereby, the insertion of HBV DNA into the vectors and its orientation are determined.

The thus obtained HBsAg gene-expression plasmids contain the HBs and HBc coding sequence in this order downstream of the phosphatase promoter. The plasmids recombined with the shuttle vectors, pAM 81, pAM 82, pAM 83 and pAM 84 are designated pAH 201, pAH 203, pAH 205 and pAH 207, respectively.

(ii) Preparation of the plasmid containing the HBsAg coding sequence

An HBsAg coding sequence (3 µg) prepared by cleaving plasmid pHBV with BamHI is treated with T4 DNA polymerase (0.2 U) in a solution (100 µl) of 67 mM Tris-HCl (pH 8.6), 6.7 mM $MgCl_2$, 10 mM 2-mercaptoethanol, 6.7 µM EDTA and 16.7 mM $(NH_4)_2SO_4$ which contains 200 µM dATP, dCTP, dTTP and dGTP for 30 minutes in order to fill in the BamHI cleavage end. The reaction mixture is subjected to phenol extraction and ethanol precipitation as described above. The resulting precipitates are subjected to a linking reaction with an Xho I linker in a molar ratio of 1:10 with T4 DNA ligase under the same conditions as described hereinbefore. After phenol extraction and ethanol precipitation, the resulting plasmid is treated with Xho I to give an HBsAg coding sequence (about 1.3 kb) having the Xho I cleavage terminus at both ends. The 1.3 kb fragment is recombined with the Xho I cleaved shuttle vector pAM 82 in a molar ratio of 5:1 by annealing and ligating with T4 DNA ligase, *E. coli* χ1776 is transformed with the reaction mixture in the same manner as described in the step 3 (i) to give plasmid DNA.

Into the plasmid thus obtained is inserted the HBsAg coding sequence in the correct orientation downstream of the phosphatase promoter of the vector pAM 82. The plasmid obtained is designated pAS 101.

(4) Preparation of transformed yeast

The starting yeast is *Saccharomyces cerevisiae* AH22 [a, leu2, his4, can1 (Cir⁺)], which has been deposited at the Fermentation Reseach Institute, Agency of Industrial Science and Technology, Japan according to the Budapest Treaty as "FERM BP-312". The starting yeast is inoculated in YPD medium (100 ml) consisting of 2% polypeptone, 1% yeast extract and 2% glucose. The mixture is incubated at 30°C overnight, and thereafter, the cells are collected by centrifugation. The cells thus collected are washed with sterilized water (20 ml), suspended in a solution (5 ml) of 1.2 M sorbitol and 100 µg/ml Zymolyase® 60,000 (manufactured by Seikagaku Kogyo K. K., Japan), and the suspension is allowed to stand at 30°C for 30 minutes to give spheroblasts. The spheroblasts thus prepared are washed with 1.2 M sorbitol solution three times, and then suspended in a solution (0.6 ml) of 2 M sorbitol, 10 mM $CaCl_2$ and 10 mM Tris-HCl (pH 7.5). The suspension thus prepared is divided into small test tubes in a volume of 60 µl. To the suspension is added the solution of the recombinant plasmid pAH 203 (30 µl) as prepared in step (3). After mixing well, 0.1 M $CaCl_2$ (3 µl) is added to a final concentration of 10 mM $CaCl_2$, and the mixture is allowed to stand at room temperature for 5 to 10 minutes. To the resulting mixture is added each 1 ml of a solution of 20% polyethylene glycol 4,000, 10 mM $CaCl_2$ and 10 mM Tris-HCl (pH 7.5), and the mixture is allowed to stand at room temperature for about 20 minutes. The resulting mixture (each 0.2 ml) is added to a medium (10 ml) consisting of 22% sorbitol, 2% glucose, 0.7% yeast nitrogen base amino acid, 2% YPD, 20 µg/ml histidine and 3% agar, which is kept at a constant temperature of 45°C. After gentle mixing, the mixture is added in a layer onto a plate of minimal medium containing 1.2 M sorbitol which is previously prepared and consists of 0.7% yeast nitrogen base amino acid, 2% glucose, 20 µg/ml histidine and 2% agar and is set thereon. The plate is incubated at 30°C to give colonies of a leucine-non-requiring yeast. The colonies are incubated in a BurkHolder minimal medium supplemented with histidine (20 µ g/ml) [cf. Tohe, A; et al; J. Bacteriol., *113*, 727—738, 1973] to give the desired transformed yeast: *Saccharomyces cerevisiae* pAH 203.

In the same manner as described above, except that the recombinant plasmids, pAS 101, pAH 201 and pAH 205 are used instead of the recombinant pAH 203, the following transformed yeasts are prepared: *Saccharomyces cerevisiae* pAS 101, *Saccharomyces cerevisiae* pAH 201, *Saccharomyces cerevisiae* pAH 205.

(5) Production of HBsAg with the transformed yeast

Each colony of the transformed yeasts obtained in step (4) is applied onto an agar plate of BurkHolder minimal medium supplemented with histidine (20 µg/ml) and incubated at 30°C to form a colony (in order to confirm the transformant requiring no leucine). The resulting cells are separated from the colony inoculated into BurkHolder minimal medium supplemented with histidine (20 µg/ml) and incubated at 30°C.

After about 24 hours, the cells in exponential growth phase are collected by centrifugation, suspended in a minimal medium (10 ml) containing no phosphoric acid (which is prepared by replacing $KH_2PO_4$ in BurkHolder minimal medium with KCl, followed by supplementing with 20 µg/ml histidine) in a cell concentration of about $4\times10^6$ cells/ml. After an incubation at 30°C for about 24 hours, the cells are collected by centrifugation of the culture broth at 4,000 r.p.m. for 10 minutes. The cells thus separated are suspended in a solution (3 ml) of 1.2 M sorbitol, 50 mM phosphate buffer (pH 7.2), 14 mM 2-mercaptoethanol and 100 µg/ml Zymolyase$^R$-60,000 (manufactured by Seikagaku Kogyo K.K. Japan), and the mixture is gently shaken at 30°C for 30 minutes to give spheroblasts. The spheroblasts are collected by centrifugation and are well suspended in a solution (1 ml) of 0.1% Tritone$^R$X-100 and 50 mM phosphate buffer (ph 7.2), stirred vigorously and then centrifuged at 7,000 r.p.m. for 10 minutes, and the resulting supernatant is taken as the yeast-lysate solution.

The lysate solution (20 µl) obtained is tested with an HBs antigen RIA kit (manufactured by Abbott, U.S.A.) for HBsAg activity. The results are shown in Table 1.

TABLE 1

| Clone No. | Host | Plasmid | HBsAg activity (cpm) |
|---|---|---|---|
| 1 | S. cerevisiae AH22 (FERM BP-312) | pAH 201 | 10,597 |
| 2 | " | pAH 203 | 13,008 |
| 3 | " | pAH 205 | 5,548 |
| 4 | " | pAS 101 | 11,200 |
| Reference | " | pAM 82* | 320 |

*) This vector contains no HBV or HBs coding sequence and is used as a negative reference.
(The negative control of the RIA kit has an activity of 310 cpm, and the positive control thereof has an activity of 17,500 cpm)

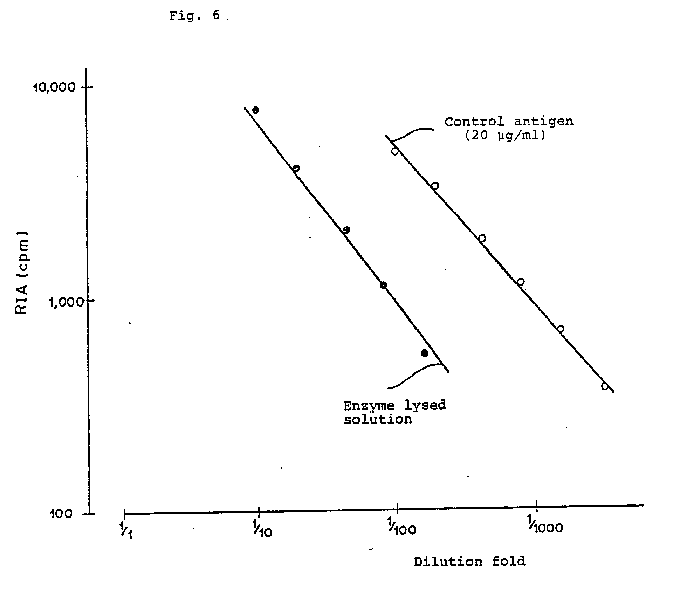

As to the yeast lysed solution (obtained above from S. cerevisiae AH22/pAH 203), the reactivity and amount of antigen are assumed in accordance with a parallel line assay using a kit for detecting HBsAg as above (cf. Finney, D. J., 1964, "Statistical method in biological assay", 2nd edition, Griffin, London), wherein purified HBsAg obtained from human blood serum is used as a control antigen. The results are shown in Figure 6. As is clear from Figure 6, the amount of antigen in the culture liquid of the transformed yeast cells is comparatively high such as 2 µg/ml. Moreover, from the parallelism with the control antigen, it is also clear that the HBsAg produced by the present invention has similar reactivities (antigenicity, immunogenicity, etc.) to those of HBsAg present in human blood plasma.

The lysed solution obtained above (each 0.4 ml) was subcutaneously inoculated into guinea pigs (female, 300—380 g, 10 animals) once a week for three weeks, and the antibodies in the blood plasma were determined with a kit for detecting anti-HBs antibodies (AUSAB, manufactured by Abbott, U.S.A.). As a result, anti-HBs antibodies were observed in all animals.

Thus, the preparation of the host vector and HBs antigen therefrom of the present invention is very useful for the production of an antigen of the Hepatitis B-inducing substance (Dane particles), and the HBs antigen prepared by the present invention is useful for the preparation of an HBV vaccine and of diagnostic reagents.

**Claims**

1. A recombinant plasmid for use in the production of the Hepatitis B virus surface antigen comprising the following components:
(a) DNA sequences of the yeast Saccharomyces cerevisiae including ars 1, the 2 µ ori, a marker gene for a transformed yeast permitting the synthesis of the leucine, histidine, tryptophane, uracil or adenine by the transformant;
(b) a DNA sequence of the E. coli plasmid pBR322 which has a size of about 3.7 kb and which includes a marker gene for a transformed E. coli encoding a resistance for ampicillin as well as the origin of replication;
(c) the expression control region of the repressible acid phosphatase gene of yeast without the entire phosphatase structural gene and without a region in the range of from +1 (ATG) to −100 bp upstream of the structural gene; and
(d) a Hepatitis B virus DNA sequence under the control of the phosphatase promoter of (c) which

9

Hepatitis B virus DNA sequence includes a fragment of 1.3 kb containing a DNA sequence coding for the 226 amino acids of the Hepatitis B virus surface antigen of the subtype adr.

2. The recombinant plasmid according to Claim 1, wherein the region to be deleted upstream of the phosphatase structural gene is in the range of from +1 to −50 bp.

3. The recombinant plasmid according to Claim 1, wherein the region to be deleted upstream of the phosphatase structural gene is in the range of from +1 to −33 bp.

4. The use of the recombinant plasmid according to any one of Claims 1 to 3 in a process for the production of Hepatitis B virus surface antigen subtype adr.

5. Process for the production of Hepatitis 3 virus surface antigen subtype adr, characterized by cultivating a yeast transformed with the recombinant plasmid according to any one of Claims 1 to 3 and isolating the antigen.

**Patentansprüche**

1. Rekombinantes Plasmid zur Verwendung bei der Herstellung von Hepatitis-B-Virus-Oberflächenantigen, umfassend die folgenden Bestandteile:

(a) DNA-Sequenzen aus der Hefe Saccharomyces cerevisiae einschließlich ars 1, dem 2 µ ori, ein Markergen, das einer transformierten Hefe die Synthese von Leucin, Histidin, Tryptophan, Uracil oder Adenin ermöglicht;

(b) eine DNA-Sequenz aus dem E. coli-Plasmid pBR322, die eine Größe von etwa 3,7 kb hat und ein für ein transformiertes E. coli Ampicillin-Resistenz codierendes Markergen ebenso wie den Ursprung der Replikation umfaßt;

(c) den Expressionskontrollbereich des reprimierbaren Gens der sauren Phosphatase von Hefe ohne das gesamte Strukturgen der Phosphatase und ohne den Bereich von +1 (ATG) bis −100 bp stromaufwärts von dem Strukturgen; und

(d) eine DNA-Sequenz von Hepatitis-B-Virus unter der Kontrolle des Promotors der Phosphatase von (c), wobei die DNA-Sequenz von Hepatitis-B-Virus ein Fragment von 1,3 kb mit einer die 226 Aminosäuren des Oberflächenantigens von Hepatitis-B-Virus, Subtyp adr, codierenden DNA-Sequenz einschließt.

2. Das rekombinante Plasmid nach Anspruch 1, wobei der stromaufwärts von dem Strukturgen der Phosphatase zu deletierende Bereich von +1 bis −50 bp reicht.

3. Das rekombinante Plasmid nach Anspruch 1, wobei der stromaufwärts von dem Strukturgen der Phosphatase zu deletierende Bereich von +1 bis −33 bp reicht.

4. Die Verwendung des rekombinanten Plasmids nach einem der Ansprüche 1 bis 3 in einem Verfahren zur Herstellung von Oberflächenantigen von Heptitis-B-Virus, Subtyp adr.

5. Verfahren zur Herstellung von Oberflächenantigen von Hepatitis-B-Virus, Subtyp adr, gekennzeichnet durch die Züchtung einer mit dem rekombinanten Plasmid nach einem der Ansprüche 1 bis 3 transformierten Hefe und Isolierung des Antigens.

**Revendications**

1. Plasmide recombinant, utile pour la production de l'antigène de surface du virus de l'hépatite B, comprenant les composants suivants:

(a) des séquences d'ADN de la levure *Saccharomyces cerevisiae*, y compris ars 1, le 2 µ ori et un gène marqueur pour une levure transformée permettant la synthèse de la leucine, de l'histidine, du tryptophane, de l'uracile ou de l'adénine par le transformant;

(b) une séquence d'ADN du plasmide pBR322 d'*E. coli* qui a une taille d'environ 3,7 kb et qui comprend un gène marqueur pour un *E. coli* transformé codant pour une résistance à l'ampicilline, ainsi que l'origine de réplication;

(c) la région de contrôle de l'expression du gène répressible de la phosphatase acide de levure, sans le gène structural entier de la phosphatase et sans une région dans l'intervalle de +1 (ATG) à −100 pb en amont du gène structural; et

(d) une séquence d'ADN du virus de l'hépatite B sous le contrôle du promoteur phosphatase de (c), laquelle séquence d'ADN du virus de l'hépatite B comprend un fragment de 1,3 kb contenant une séquence d'ADN codant pour les 226 amino-acides de l'antigène de surface du virus de l'hépatite B du sous-type adr.

2. Plasmide recombinant selon la revendication 1, dans lequel la région à supprimer en amont du gène structural de la phosphatase est dans la gamme de +1 à −50 pb.

3. Plasmide recombinant selon la revendication 1, dans lequel la région à supprimer en amont du gène structural de la phosphatase est dans la gamme de +1 à −33 pb.

4. Utilisation du plasmide recombinant selon l'une quelconque des revendications 1 à 3 dans un procédé pour la production du sous-type adr de l'antigène de surface du virus de l'hépatite B.

5. Procédé pour la production du sous-type adr de l'antigène de surface du virus de l'hépatite B, caractérisé par la culture d'une levure transformée avec le plasmide recombinant selon l'une quelconque des revendications 1 à 3 et l'isolement de l'antigène.

Fig. 1

Xho I     HBs gen*e*        Bam HI    HBc gen*e*        Xho I

27bp

Fig. 3

EcoRI
*ars* I
Apr
2μm
*Leu*2
pAT77
EcoRI
EcoRI
EcoRI
BamHI
ATG
Sal I

1. Sal I
2. Bal 31
3. XhoI linker
    T4 DNA ligase

EcoRI
*ars* I
Apr
2μm
*Leu*2
pAM82
EcoRI
EcoRI
Bam HI
XhoI

Fig. 4

Bst EII     Hogness box      acidic phosphatase structural gene    Sal I

-100        ATG +1        +82   pBR322

1

Fig. 2(1)

Peptide 3

Peptide 4

..CCTGCACCGAACATG GAG AAC ACA ACA TCA GGA TTC CTA GGA CCC CTG CTC GTG TTA CAG GCG GGG TTT TTC TTG TTG ACA AGA ATC CTA ACA ATA CCA CAG

subtype adr → Met-Glu-Asn-Thr-Thr-Ser-Gly-Phe-Leu-Gly-Pro-Leu-Leu-Val-Leu-Gln-Ala-Gly-Phe-Phe-Leu-Leu-Thr-Arg-Ile-Leu-Phr-Ile-Pro-Gln-

subtype ad/yw →       Ile

subtype adw →       Ile

subtype ayw →       Ile

Peptide 4

Peptide 1

AGT CTA GAC TCG TGG TGG ACT TCT CTC AAT TTT CTA GGG GGA GCA CCC ACG TGT CCT GGC CAA AAT TCG CAG TCC CCA ACC TCC AAT CAG

Ser-Leu-Asp-Ser-Trp-Trp-Thr-Ser-Leu-Asn-Phe-Leu-Gly-Gly-Ala-Pro-Thr-Cys-Pro-Gly-Gln-Asn-Ser-Gln-Ser-Pro-Thr-Ser-Asn-His

           Thr Thr Val    Leu                  Ile

           Ser Pro Val    Leu                  Thr

           Thr Thr Val    Leu                  Thr

Hydrophilic
Cys-rich
region

Peptide 1

TCA CCA ACC TCT TGT CCT CCA ATT TGT CCT GGC TAT CGC TGG ATG TGT CTG CGG CGT TTT ATC ATA TTC CTC TTC ATC CTG CTG CTA TGC

Ser-Pro-Thr-Ser-Cys-Pro-Pro-Ile-Cys-Pro-Gly-Thr-Arg-Trp-Met-Cys-Leu-Arg-Arg-Phe-Ile-Ile-Phe-Ile-Phe-Ile-Leu-Leu-Leu-Cys

           Thr

           Ile

           Thr

Hydrophobic region

EP 0 105 149 B1

Fig. 2(2)

Peptide 6

100                  110             120

CTC ATC TTC TTG TTG GTT CTT CTG GAC TAC CAA GGT ATG TTG CCC GTT TGT CCT CTA CTT CCA GGA ACA TCA ACT ACC AGC ACG GGA CCA

Leu-Ile-Phe-Leu-Leu-Val-Leu-Leu-Asp-Tyr-Gln-Gly-Met-Leu-Pro-Val-Cys-Pro-Leu-Leu-Pro-Gly-Thr-Ser-Thr-Thr-Ser-Thr-Gly-Pro

Hydrophobic region

| | | | | |
|---|---|---|---|---|
| Ile | Ser Ser | | | Ser |
| Ile | Ser Thr | | | Pro |
| Ile | Ser Ser | | | Pro |

Hydrophilic Cys-rich region

130                  140             150

TGC AAG ACC TGC ACG ATT CCT GCT CAA GGA ACC TCT ATG TTT CCC TCT TGT TGC TGT ACA AAA CCT TCG GAC GGA AAC TGC ACT TGT ATT

Sys-Lys-Thr-Cys-Thr-Ile-Pro-Ala-Gln-Gly-Thr-Ser-Met-Phe-Pro-Ser-Cys-Cys-Cys-Thr-Lys-Pro-Ser-Asp-Gly-Asn-Cys-Thr-Cys-Ile

| | | | | | |
|---|---|---|---|---|---|
| Arg | | Thr | Ile | Tyr | Ser |
| Lys | | Thr | Asn | Phe | Thr |
| Arg | Met | Thr | Thr | Tyr | Ser |

160                  170             180

CCC ATC CCA TCA TCC TGG GCT TTC GCA AGA TTC CTA TGG GAG GGG GCC TCA GTC CGT TTC TCC TGG CTC AGT TCA CTA GTG CCA TTT GTT

Pro-Ile-Pro-Ser-Ser-Trp-Ala-Phe-Ala-Arg-Phe-Leu-Trp-Glu-Gly-Ala-Ser-Val-Arg-Phe-Ser-Trp-Leu-Ser-Leu-Leu-Val-Pro-Phe-Val

Hydrophobic region

| | | | | |
|---|---|---|---|---|
| Gly Lys Phe | | | Trp | Ala |
| Ala Lys Tyr | | | Trp | Val |
| Gly Lys Phe | | | Trp | Ala |

EP 0 105 149 B1

EP 0 105 149 B1

Fig. 2(3)

190 200 210
CAG TGG TTC GTA GGG CTT TCC CCC ACT GTT TGG CTT TCA GTT ATA TGG ATG ATG TGG TAT TGG GGA CCA AGT CTG TAC AAC ATC TTG AGT

Gln-Trp-Phe-Val-Gly-Leu-Ser-Pro-Thr-Val-Trp-Leu-Ser-Val-Ile-Trp-Met-Met-Trp-Tyr-Trp-Gly-Pro-Ser-Leu-Tyr-Asn-Ile-Leu-Ser

Ile            Val                                              Ser    Leu

Thr            Ala                                              Ser    Val

Thr            Val                                              Ser    Leu

Hydrophobic
region

220 226
CCC TTT TTA CCT CTA TTA CCA ATT TTC TTT TGT CTT TGG GTA TAC ATT TAA ACCCTAATAAAACCAAACGTTGGGGCTACTCCCTTAACTTCATGGGATATGTAAT...

Pro-Phe-Leu-Pro-Leu-Leu-Pro-Ile-Phe-Phe-Cys-Leu-Trp-Val-Tyr-Ile

Leu                                        Ala

Fig. 5

Bst EII
↓
GTCACCTTACTTGGCAAGGCATATACCCATTTGGGATAAAGGGTAAACACTT
-170   -160   -150   -140   -130

TGAATT(G)TCGAAAATGAAACGTATATAAGACGCTGATGTTTTGCTAAGTCGAGGTTAGTATGGCTTCATCTCTCA
-120  -110  -100  -90  -80  -70  -60  -50

TGAGAATAAGAACAACAACAAATAGAGCAAGCAAATTCGAGATTACCAATG........GTCGAC +82
-40   -30   -20   -10   +1                              Sal I

Fig. 6 .